(19) Europäisches Patentamt

European Patent Office

Office européen des brevets

(11) **EP 4 265 731 A1**

(12) **EUROPEAN PATENT APPLICATION**

(43) Date of publication:
**25.10.2023 Bulletin 2023/43**

(21) Application number: **22168828.6**

(22) Date of filing: **19.04.2022**

(51) International Patent Classification (IPC):
***C12Q 1/44*** *(2006.01)*

(52) Cooperative Patent Classification (CPC):
**C12Q 1/44;** G01N 2333/918; G01N 2400/40;
G01N 2500/00

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicant: **Biofer S.p.A.**
**20121 Milano (MI) (IT)**

(72) Inventors:
• **MORANDI, Riccardo**
**41013 CASTELFRANCO EMILIA (MODENA) (IT)**

• **FERRARESI, Roberta**
**41031 CAMPOSANTO (MODENA) (IT)**
• **BANDIERA, Elisa**
**44012 BONDENO (FERRARA) (IT)**
• **TALASSI, Mara**
**41034 FINALE EMILIA (MODENA) (IT)**

(74) Representative: **Biggi, Cristina**
**Bugnion S.p.A.**
**Viale Lancetti 17**
**20158 Milano (IT)**

(54) **METHOD FOR TESTING DRUGS**

(57) The present invention relates to an in vitro method for evaluating the lipasaemic activity of a glycosaminoglycan-based compound comprising a heparin fraction and optionally dermatan sulphate. Said method comprises at least a step of placing in contact, with a lipid substrate, a plurality of biological samples isolated from at least two groups of individuals to which the compound had been administered in order to obtain plasma-substrate systems.

EP 4 265 731 A1

**Description**

**FIELD OF THE INVENTION**

The present invention relates to an in vitro method for evaluating the lipasaemic activity of a compound.

**PRIOR ART**

The activity of testing drugs is focused on demonstrating that different lots of a medicine produced over time are always in compliance with what was authorised by the competent authority at the time of issue of the Marketing Authorisation (MA) to the manufacturer.

This testing is carried out through the evaluation of various aspects, such as, for example, a verification of production conditions (observance of Good Manufacturing Practices - GMP - in the pharmaceutical factory) and the conducting of numerous analytical tests on the medicine and some of its processing intermediates.

Manufacturers test new lots of drugs before placing them on the market to verify their conformity.

Sulodexide is an active substance of biological origin and thus requires a verification of the individual lots to be placed on the market. Sulodexide is a substance with an antithrombotic action and performs its action by interacting at the level of the coagulation process with different factors of the coagulation cascade, in particular, with the activated factor X. The antithrombotic action of Sulodexide is also sustained by the inhibition of platelet aggregation and the activation of the fibrinolytic system. Furthermore, through the activation of lipoprotein lipase it is effective in normalising altered lipid levels. The specific release tests on Sulodexide include the determination of its lipasaemic activity, a biological assay performed on animals, for example rats.

The lipasaemic activity of Sulodexide is its capacity to release the enzyme lipoprotein lipase into the blood, which hydrolyses the triglycerides of lipoproteins. As it is an in vivo assay, hence performed on animals, it is important that it be reliable and reproducible, and at the same time it must involve the least number of animals possible. Furthermore, the specificity of the assay is of great relevance in order to be able to determine the conformity of new lots of Sulodexide to be placed on the market.

**SUMMARY OF THE INVENTION**

A first aspect of the present invention relates to an in vitro method for evaluating the lipasaemic activity of a glycosaminoglycan-based compound comprising a heparin fraction and optionally dermatan sulphate. Preferably, said method preferably comprises at least a step of placing in contact, with a lipid substrate, a plurality of biological samples isolated from at least a first group of individuals to which a first concentration of the compound had been administered and from at least a second group of individuals to which a second concentration of the compound, different from the first concentration, had been administered, in order to obtain plasma-substrate systems obtained from the tested sample.

Preferably, after the step of placing in contact, the optical density of the plasma-substrate systems obtained from the tested sample is read at a wavelength comprised between 550 and 650 nm, preferably at about 600 nm, after a time comprised between 0 and 2 minutes (T0) and after a time (Tf) comprised between 15 and 40 minutes from the placing in contact, in order to determine the percentage reduction in the optical density of the plasma-substrate systems obtained from the tested sample. The lipasaemic activity of the compound is preferably determined by interpolating the reduction in the optical density of the plasma-substrate systems obtained from the tested sample with a standard curve obtained with a compound with known lipasaemic activity (standard compound).

The lipid substrate and the compound are preferably placed in contact with each other at a temperature comprised between 36 and 40°C, preferably at about 37-38°C.

**DEFINITIONS**

In the context of the present invention "conforming to specifications" means a lipasaemic activity of a compound that is comprised in a range of acceptability established by a pharmaceutical company.

**DETAILED DESCRIPTION OF THE INVENTION**

A first aspect of the present invention relates to an in vitro method for evaluating the lipasaemic activity of a glycosaminoglycan-based compound comprising a heparin fraction and optionally dermatan sulphate. In one embodiment, said method comprises at least a step of placing in contact, with a lipid substrate, a plurality of biological samples isolated from at least a first group of individuals to which a first concentration of the compound had been administered and from at least a second group of individuals to which a second concentration of the compound, different from the first concentration, had been administered, in order to obtain plasma-substrate systems obtained from the tested sample.

Preferably, after the step of placing in contact, the optical density of the plasma-substrate systems obtained from the tested sample is read at a wavelength comprised between 550 and 650 nm, preferably at 600 nm, after a time comprised between 0 and 2 minutes (T0) and after a time (Tf) comprised between 15 and 40 minutes from the placing in contact, in order to determine the percentage reduction in the optical density of the plasma-substrate systems obtained from the tested sample. Preferably, after the percentage reduction in the plasma-substrate systems obtained from the tested sample has been obtained, they are interpolated into a standard curve in order to obtain the lipasaemic activity of the compound.

In one embodiment, the method comprises the steps of:

a) providing a lipid substrate;
b) placing in contact with the lipid substrate:

- a plurality of biological samples isolated from a first group of individuals to which a first concentration of the compound had been administered,
- a plurality of biological samples isolated from a second group of individuals to which a second concentration of the compound, different from the first concentration of the compound, had been administered, and
- optionally, a plurality of biological samples isolated from a third group of individuals to which a third concentration of the compound, different from the first and second concentrations of the compound, had been administered, in order to obtain at least four plasma-substrate systems obtained from the tested sample;

c) placing in contact with the lipid substrate:

- a plurality of biological samples isolated from a fourth group of individuals to which a first concentration of the standard compound had been administered,
- a plurality of biological samples isolated from a fifth group of individuals to which a second concentration of the standard compound, different from the first concentration of the standard compound, had been administered, and
- optionally, a plurality of biological samples isolated from a sixth group of individuals to which a third concentration of the standard compound, different from the first and second concentrations of the standard compound, had been administered, in order to obtain at least four plasma-substrate systems obtained from the reference standard;

d) reading the optical density of the plasma-substrate systems obtained in the steps b) and c) at a wavelength comprised between 550 and 650 nm, preferably at about 600 nm, after a time comprised between 0 and 2 minutes (T0) from the start of step b) and of step c);
e) reading the optical density of the plasma-substrate systems obtained in steps b) and c) at a wavelength comprised between 550 and 650 nm, preferably at about 600 nm, after a time comprised between 15 and 40 minutes (Tf) from the start of step b) and of step c);
f) evaluating the percentage reduction in the optical density (A%) of each plasma-substrate system obtained from the tested sample and of each plasma-substrate system obtained from the reference standard according to the following formula:

$$A\% = (\text{Absorbance Tf} / \text{Absorbance T0}) \times 100.$$

In one embodiment, the A% values of the plasma-substrate systems obtained from the reference standard are used to prepare a standard dose-response curve, wherein the dose refers to the concentration in LSU/ml of the standard compound administered and the response is the A%.

The A% values of the plasma-substrate systems obtained from the tested sample are preferably used to prepare a dose-response curve wherein the dose is the concentration in LSU/ml of the compound administered and the response is the A%.

In one embodiment, the lipasaemic activity of the compound is extrapolated by interpolating the A% from the dose-response curve of the sample with the A% of the standard dose-response curve.

In other words, as the reference standard has a known lipasaemic activity, the A% are interpolated with the A% of the reference standard and the lipasaemic activity in LSU/mg of the tested sample is derived.

In one embodiment, the extrapolation of the lipasaemic activity from the standard curve is performed using a statistical model known to the person skilled in the art. Said statistical model preferably takes into consideration the slope of the standard curve and of the dose-response curve of the tested sample, and the presumed lipasaemic activity of the tested sample. In a preferred embodiment of the invention, said statistical model is the parallel-line analysis (PLA) method according to chapter 5.3 of the *European Pharmacopoeia* 10.0.

In a preferred embodiment of the invention, each group of individuals to which the compound or standard compound had been administered comprises at least two individuals; it preferably comprises a number of individuals comprised between four and eight, more preferably comprised between five and six individuals.

In one embodiment, the plurality of biological samples isolated for each group comprises at least two samples; it preferably comprises a number of samples comprised between four and eight, more preferably comprised between five and six.

The compound and the standard compound preferably comprise both a heparin fraction and dermatan sulphate. Preferably, the compound and the standard compound each comprise a larger amount of heparin fraction than dermatan sulphate. In one embodiment, said compound and said standard compound comprise a percentage comprised between 35 and 95% of heparin fraction, preferably comprised between 50 and 85%. In one embodiment, in addition to the heparin fraction, the compound and the standard compound comprise dermatan sulphate in the amount necessary to arrive at 100% of the compound.

In a preferred embodiment of the invention, the compound and the standard compound are Sulodexide.

The standard compound is preferably a compound whose known lipasaemic activity is comprised between 8 and 16 LSU/mg, preferably comprised between 10 and 14 LSU/mg.

In embodiment, the compound and the standard compound were administered to the individuals by parenteral administration, preferably by injection, more preferably by injection into the caudal vein. In a preferred embodiment, the individuals are animals, preferably rats.

In one embodiment, the biological samples are blood samples and were isolated from the individuals by drawing blood from an artery, preferably from the abdominal aorta of the individual.

Preferably, the biological samples, more preferably the blood samples, are treated with techniques known to the person skilled in the art, in order to obtain plasma. For example, the plasma is obtained by density gradient centrifugation of the biological samples. Therefore, the biological sample that is placed in contact with the lipid substrate is preferably plasma.

In one embodiment, the lipasaemic activity of the compound is deemed to conform to specifications if it is comprised between 10 and 16 LSU/mg, preferably if it is comprised between 10 and 14 LSU/mg. In other words, if the lipasaemic activity of the compound is comprised between 10 and 14 LSU/mg, the compound is deemed to conform to specifications.

In one embodiment, step a) comprises the following sub-steps:

   a1) preparing an aqueous phase;
   a2) preparing an oily phase;
   a3) mixing the aqueous phase obtained in sub-step a1) with the oily phase obtained in sub-step a2) using a homogeniser to obtain the lipid substrate; and
   a4) optionally sonicating the lipid substrate obtained in step a3).

Preferably, the aqueous phase comprises an anticoagulant agent, preferably ethylenediaminetetraacetic acid (EDTA), at least one sugar, preferably a disaccharide, more preferably sucrose. In one embodiment, the aqueous phase further comprises at least one preservative and/or at least one surfactant.

Preferably, the oily phase comprises an oily matrix, more preferably selected from among coconut oil, maize oil, cottonseed oil, mineral oil, soybean oil, castor oil and combinations thereof.

In one embodiment, the oily phase further comprises at least one preservative and/or at least one antioxidant.

In step a3) the mixing of the oily phase with the aqueous phase preferably takes place at a temperature comprised between 35 and 75°C, preferably between 40 and 65°C, for a time comprised between 2 and 5 hours, more preferably about 3-4 hours.

In one embodiment, the lipid substrate obtained is solubilised in a solvent, preferably in water, more preferably in ultra-pure water. The lipid substrate is preferably solubilised in the solvent to obtain a concentration comprised between 0.25 and 2% w/w of lipid substrate diluted in the solvent, more preferably comprised between 0.4 and 1.25% w/w.

In one embodiment, in step a4), the lipid substrate is sonicated for a time comprised between 5 and 20 minutes, preferably comprised between 10 and 18 minutes at a temperature comprised between 30 and 40°C, preferably at about 35-38°C.

In one embodiment, steps b) and c) are carried out at a temperature comprised between 36 and 40°C, preferably at about 37-38°C. In other words, steps b) and c) are carried out at a temperature that is very close to the body temperature of the individual, preferably a rat. In this manner, the body environment is mimicked and the reaction between the compound and the lipid substrate takes place under physiological-like conditions. The individuals from whom the biological samples used in step b) were isolated have preferably received an amount of a compound comprised between 1 and 10 lipasaemic units/ml (LSU/ml).

The individuals have preferably received a concentration of a compound or of a standard compound, preferably Sulodexide, comprised between 1 and 10 LSU/ml.

The first concentration of the compound and of the standard compound is preferably comprised between 0.5 and 4 LSU/ml, more preferably comprised between 1 and 3 LSU/ml; the second concentration of the compound and of the standard compound is comprised between 1 and 6 LSU/ml, more preferably comprised between 2 and 5 LSU/ml; and the third concentration of the compound and of the standard compound is comprised between 4 and 10 LSU/ml, more preferably comprised between 6 and 9 LSU/ml.

In one embodiment, the above-described concentrations of the compound and of the standard compound were administered in a volume comprised between 0.5 and 2 ml per individual, preferably comprised between 0.8 and 1.2 ml.

The Applicant has devised a method that enables a specific, repeatable and reliable evaluation of the lipasaemic activity of a compound comprising a heparin fraction and optionally dermatan sulphate. In fact, as shown in the example, the method enables a specific analysis of the lipasaemic activity of samples taken from individuals to which the compound had been administered. The preparation of the compound, thanks to the use of a homogeniser and a sonicator, enables a homogeneous solution and a very linear percentage reduction in absorbance (A%) to be obtained. In addition, the concentrations of the compound and standard compound administered make it possible to obtain higher percentage reductions in absorbance compared to the known methods, with a better linearity of the dose-response curves obtained.

Finally, the biological samples isolated with the substrate are incubated at a temperature of about 37-38°C in order to mimic the activity of the enzyme lipase in vivo, enabling a reaction under conditions very similar to the physiological ones of the animal.

A **second** aspect of the present invention relates to a method for evaluating the lipasaemic activity of a glycosaminoglycan-based compound comprising a heparin fraction and optionally dermatan sulphate. In one embodiment, said method comprises at least a step of placing in contact, with a lipid substrate, a plurality of biological samples isolated from at least a first group of individuals to which a first concentration of the compound is administered and from at least a second group of individuals to which a second concentration of the compound, different from the first concentration, is administered, in order to obtain plasma-substrate systems obtained from the tested sample.

Preferably, after the step of placing in contact, the optical density of the plasma-substrate systems obtained from the tested sample is read at a wavelength comprised between 550 and 650 nm after a time comprised between 0 and 2 minutes (T0) and after a time (Tf) comprised between 15 and 40 from the placing in contact, in order to determine the percentage reduction in the optical density of the plasma-substrate systems obtained from the tested sample.

In one embodiment, the method comprises the steps of:

a) administering a first concentration of the compound to a first group of individuals, a second concentration of the compound to a second group of individuals and, optionally, a third concentration of the compound to a third group of individuals;

b) administering a first concentration of a compound with a known lipasaemic activity (LSU/mg) (standard compound) to a fourth group of individuals, a second concentration of the standard compound to a fifth group of individuals and optionally, a third concentration of the standard compound to a sixth group of individuals;

c) isolating at least one biological sample from each individual to which the compound in step a) was administered;

d) isolating at least one biological sample from each individual to which the standard compound in step b) was administered;

e) providing a lipid substrate;

f) placing in contact the biological samples isolated in step c) with the lipid substrate in order to obtain at least four plasma-substrate systems obtained from the tested sample;

g) placing the biological samples isolated in step d) in contact with the lipid substrate in order to obtain at least four plasma-substrate systems obtained from the reference standard;

h) reading the optical density of the plasma-substrate systems obtained from the tested sample and of the plasma-substrate systems obtained from the reference standard in steps f) and g) at a wavelength comprised between 550 and 650 nm, preferably at about 600 nm, after a time comprised between 0 and 2 minutes (T0) from the start of step f) and of step g);

i) reading the optical density of the plasma-substrate systems obtained from the tested sample and of the plasma-substrate systems obtained from the reference standard in steps f) and g) at a wavelength comprised between 550 and 650 nm, preferably at about 600 nm, after a time comprised between 15 and 40 minutes (Tf) from the start of step f) and of step g);

l) evaluating the percentage reduction in the optical density (A%) of each plasma-substrate system obtained from the tested sample and of each plasma-substrate system obtained from the reference standard according to the following formula:

$$A\% = (Absorbance\ Tf\ /\ Absorbance\ T0)\ X\ 100.$$

In one embodiment, the A% values of the plasma-substrate systems obtained from the reference standard are used to prepare a standard dose-response curve, wherein the dose refers to the concentration in LSU/ml of the standard compound administered and the response is the A%.

The A% values of the plasma-substrate systems obtained from the tested sample are preferably used to prepare a dose-response curve wherein the dose is the concentration in LSU/ml of the compound administered and the response is the A%.

In one embodiment, the lipasaemic activity of the compound is extrapolated by interpolating the A% from the dose-response curve of the sample with the A% of the standard dose-response curve.

In other words, as the reference standard has a known lipasaemic activity, the A% of the plasma-substrate systems are interpolated with the A% of the plasma-substrate systems obtained from the reference standard and the lipasaemic activity n LSU/mg of the tested sample is derived.

In one embodiment, the extrapolation of the lipasaemic activity from the standard curve is performed using a statistical model known to the person skilled in the art. Said statistical model preferably takes into consideration the slope of the standard curve and of the dose-response curve of the tested sample, and the presumed lipasaemic activity of the sample to be tested.

In a preferred embodiment of the invention, said statistical model is the parallel-line analysis (PLA) method according to chapter 5.3 of the *European Pharmacopoeia* 10.0.

In a preferred embodiment of the invention, each group of individuals to which the sample or the sample standard had been administered comprises at least two individuals; it preferably comprises a number of individuals comprised between four and eight, more preferably comprised between five and six individuals.

The compound and the standard compound preferably comprise both a heparin fraction and dermatan sulphate. In one embodiment, the compound and the standard compound each comprise a greater amount of heparin fraction than dermatan sulphate. In one embodiment, said compound and said standard compound comprise a percentage comprised between 35 and 95% of heparin fraction, preferably comprised between 50 and 85%. In one embodiment, in addition to the heparin fraction, the compound and the standard compound comprise dermatan sulphate in the amount necessary to arrive at 100% of the compound.

In a preferred embodiment of the invention, the compound and the standard compound are Sulodexide.

The standard compound is preferably a compound whose known lipasaemic activity is comprised between 8 and 16 LSU/mg, preferably comprised between 10 and 14 LSU/mg.

In one embodiment, the compound and the standard compound are administered to the individuals by parenteral administration, preferably by injection. In a preferred embodiment, the individuals are animals, preferably rats; the compound and the standard compound, more preferably Sulodexide, are preferably administered by injection into the caudal vein.

In one embodiment, the biological samples are blood samples and are isolated from the individual by drawing blood from an artery, preferably from the abdominal aorta of the individual.

The biological samples, more preferably the blood samples, are preferably treated with techniques known to the person skilled in the art, in order to obtain plasma. For example, the plasma is obtained by density gradient centrifugation of the biological samples. Therefore, the biological sample that is placed in contact with the lipid substrate is preferably plasma.

In one embodiment, the lipasaemic activity of the compound is deemed to conform to specifications if it is comprised between 10 and 16 LSU/mg, preferably if it is comprised between 10 and 14 LSU/mg. In other words, if the lipasaemic activity of the compound is comprised between 10 and 14 LSU/mg, the compound is deemed to conform to specifications.

In one embodiment, step a) comprises the following sub-steps:

a1) preparing an aqueous phase;
a2) preparing an oily phase;
a3) mixing the aqueous phase obtained in sub-step a1) with the oily phase obtained in the sub-step a2) using a homogeniser to obtain the lipid substrate; and
a4) optionally sonicating the lipid substrate obtained in step a3).

The aqueous phase preferably comprises an anticoagulant agent, preferably ethylenediaminetetraacetic acid (EDTA), at least one sugar, preferably a disaccharide, more preferably sucrose. In one embodiment, the aqueous phase further comprises at least one preservative and/or at least one surfactant.

The oily phase preferably comprises an oily matrix, more preferably selected from among coconut oil, maize oil, cottonseed oil, mineral oil, soybean oil, castor oil and combinations thereof.

In one embodiment, the oily phase further comprises at least one preservative and/or at least one antioxidant.

The mixing of the oily phase with the aqueous phase preferably takes place at a temperature comprised between 35 and 75°C, preferably between 40 and 65°C, for a time comprised between 2 and 5 hours, more preferably about 3-4 hours.

In one embodiment, the lipid substrate obtained is solubilised in a solvent, preferably in water, more preferably in ultra-pure water. The lipid substrate is preferably solubilised in the solvent to obtain a concentration comprised between 0.25 and 2% w/w of lipid substrate diluted in the solvent, more preferably comprised between 0.4 and 1.25% w/w.

In one embodiment, in step a4), the lipid substrate is sonicated for a time comprised between 5 and 20 minutes, preferably comprised between 10 and 18 minutes, at a temperature comprised between 30 and 40°C, preferably at about 35-38°C.

In one embodiment, steps f) and g) are carried out at a temperature comprised between 36 and 40°C, preferably at about 37-38°C. In other words, steps f) and g) are carried out at a temperature that is very close to the body temperature of the individual, preferably a rat. In this manner, the body environment is mimicked and the reaction between the compound and the lipid substrate takes place under physiological-like conditions. The individuals from whom the biological samples used in step f) are isolated preferably receive a concentration of the compound comprised between 1 and 10 lipasaemic units/ml (LSU/ml).

The first concentration of the compound and of the standard compound is comprised between 0.5 and 4 LSU/ml, more preferably comprised between 1 and 3 LSU/ml; the second concentration of the compound and of the standard compound is preferably comprised between 1 and 6 LSU/ml, more preferably comprised between 2 and 5 LSU/ml; and the third concentration of the compound and of the standard compound is comprised between 4 and 10 LSU/ml, more preferably comprised between 6 and 9 LSU/ml.

In one embodiment, the above-described concentrations of the compound and of the standard compound are administered in a volume comprised between 0.5 and 2 ml per individual, preferably comprised between 0.8 and 1.2 ml.

## EXAMPLE

### Preparation of the lipid substrate

### Preparation of oily phase:

12.5 g of coconut oil were weighed in a 40 ml glass beaker and placed on a hot plate so as to bring the solution to 60°C (maintenance of the temperature was verified by means of a calibrated thermometer).

The solution was placed in a homogeniser; 2.5 mg of butylated hydroxyanisole and 0.375 g of monolauryl glycerol were added. The solution was homogenised for 30 minutes.

### Preparation of aqueous phase:

The following were weighed in a 20 ml glass beaker:

- 0.5 g of Tween 80,
- 0.05 g of methyl p-hydroxybenzoate,
- 3.125 g of sucrose,
- 12.5 mg of ethylenediaminetetraacetic acid disodium salt.

The components were solubilised in 12.5 ml of purified water and placed on a stirrer plate until complete dissolution.

### Mixing of the two phases:

The aqueous phase was added to the oily phase, maintaining the homogeniser in action and maintaining the solution at a temperature of 60°C for three hours.

The hot plate was turned off and the homogeniser was left in action for an hour; after this time elapsed, the solution was allowed to cool completely, before being transferred into a refrigerator.

The lipid substrate thus obtained appeared as a creamy white solid.

Testing of the lipid substrate

The lipid substrate was solubilised in ultra-pure water so as to obtain a solution with a concentration comprised between 0.5 and 1.0%. The solution was sonicated for 15' at a temperature of 37°C; after this time elapsed, it was placed under continuous stirring using a magnetic stirrer. 2 ml of saline solution and 1 ml of lipid substrate were placed in a cuvette; the absorbance of this solution was tested at a wavelength of 600 nm after zeroing the instrument against air.

The reading had to be comprised between 0.725 and 0.775 UAbs. If the absorbance did not fall in the required range, a new dilution of the lipid substrate was performed.

Preparation of the standard solutions and of the sample to be tested

Standard solution: a pre-weighed amount of Sulodexide Reference Standard had a volume of saline solution added to it in order to obtain a solution at 40 LSU/ml (solution S) based on the certified activity. The solution was shaken in a vortex mixer until dissolving completely (Solution S).

Example: 100 mg of Sulodexide RS, having an activity of 12 LSU/mg, were weighed to obtain a 40 LSU/ml solution according to the following calculation:

100 mg x 12 LSU/mg = 1200 LSU to obtain a solution at 40 LSU/ml
1200 LSU/40 LSU/ml = 30 ml of saline solution to be added to obtain a solution at 40 LSU/ml
**Solution S:** 40 LSU/ml
**Solution A** (8 LSU/ml): 4 ml of solution S were dispensed into a 50 ml disposable test tube and 16 ml of saline solution were added.
**Solution B** (4 LSU/ml): 10 ml of solution A were dispensed into a 50 ml disposable test tube and 10 ml of saline solution were added.
**Solution C** (2 LSU/ml): 10 ml of solution B were dispensed into a 50 ml disposable test tube and 10 ml of saline solution were added.

**Test solution:** about 100-150 mg of the Sulodexide sample were weighed and placed in a disposable test tube; a volume of saline solution was added so as to obtain a solution at 40 LSU/ml (solution T) based on the known activity of 12 LSU/mg (median relative to the limits of 10-14 LSU/mg). The solution was shaken in a vortex mixer until dissolving completely. (Solution T). In order calculate the volume of saline solution to be added, the procedure indicated in the example regarding preparation of the standard was followed.

**Solution T:** 40 LSU/ml
**Solution D** (8 LSU/ml): 4 ml of solution T were dispensed into a 50 ml disposable test tube and 16 ml of saline solution were added.
**Solution E** (4 LSU/ml): 10 ml of solution D were dispensed into a 50 ml disposable test tube and 10 ml of saline solution were added.
**Solution F** (2 LSU/ml): 10 ml of solution E were dispensed into a 50 ml disposable test tube and 10 ml of saline solution were added.

For each standard and sample solution (2 LSU/ml, 4LSU/ml and 8 LSU/ml), 6 Eppendorf micro test tubes were prepared, into which 1060 µl of the solution were dispensed so as to allow 1 ml of the drug to be drawn and subsequently administered.

Housing of the animals

The animals had a body weight comprised between 175 and 200 grams. There was a 5-day wait before the test so that the animals could acclimatise.

The animals were weighed the day before the test to ensure that they had a weight comprised between 180 and 220 grams and they were examined by a veterinarian to verify their state of health.

Throughout the period they were housed in the facility, the animals were fed ad libitum with a standard diet "Mucedola 4RF21 Certificate-PF-1610-for mice and rats" and drinking water.

Treatment of the animals

1 ml of a reference solution was injected into the lateral caudal vein of the rats, while the animal was awake (restrained by an operator); at the end of administration the animal was placed back into its cage. Six animals were used for every concentration of the standard and sample.

The scheme is summarised below in Table 1:

Table 1

| *Substance* | *Concentration in LSU/ml* | *Number of animals* |
|---|---|---|
| Sulodexide (Standard) | 2 | 6 |
| | 4 | 6 |
| | 8 | 6 |
| Sulodexide (Sample) | 2 | 6 |
| | 4 | 6 |
| | 8 | 6 |
| Saline solution (placebo)* | N.A. | 1 |
| * Placebo to be administered only at the validation stage. | | |

Blood sample collection and euthanasia

Anaesthesia of the animals was induced in an induction chamber by means of a gas anaesthetic (IsoFlo, Zoetis Italia, Rome), 3% isoflurane in 1 litre of oxygen.

Once the surgical level of anaesthesia was confirmed by pinching the abdomen, base of the tail and rear paw, the animals were positioned supine on the operating table, while anaesthesia was always maintained with a face mask. An incision was then made in the abdomen to enable access to the abdominal aorta, from which about 5 ml of blood were drawn using 10 ml syringes with a 20G needle.

N.B. in the event that a problem occurred during the sample collection, e.g. rupture of the aorta (with the consequent impossibility of collecting blood), or the blood drawn was insufficient, it was possible to use a new animal to enable a new administration and sample collection.

The use of at most three additional animals was allowed per work session. At the end of the sample collection haemostasis was not induced; the animal was allowed to pass from deep anaesthesia to death.

There was a 10-minute wait between the time the administration ended and the start of the sample collection.

Management of biological fluids

Immediately after being collected, the blood was transferred into Vacumed 42482 test tubes (test tubes pre-filled with sodium citrate as an anticoagulant), numbered with the animal's ID. The test tube contents were carefully mixed by inversion.

Immediately after a sample had been collected from the last animal of every treatment group (groups made up of 6 animals, with the exception of the placebo), the test tubes were centrifuged at 3900 xg for a time of 20' (acceleration set on 8, deceleration set on 1).

The plasma thus obtained was drawn gently using of a mechanical pipette (taking care not to draw in the corpuscular component) and transferred into a new test tube marked with the animal's ID and placed in a refrigerator at a controlled temperature until the time of reading.

Spectrophotometric readings

2 ml of plasma obtained were dispensed into a disposable cuvette and 1 ml of lipid substrate was added, at a controlled temperature of 38°C.

The optical density of the "plasma-substrate system" was read with a UV spectrophotometer at a wavelength of 600 nm after 1' (T0) and after 15' of incubation (Tf).

For each "plasma-substrate system" the percentage reduction (A%) in the optical density was evaluated according to the following formula:

**A% = (Abs Tf / Abs T0) X 100**

Using a specific spreadsheet, the regression of the response (A%) was calculated against the natural logarithm of the concentrations of the substance to be examined and of the reference standard of Sulodexide and the activity of the sample was then calculated using the statistical methods of parallel-line analysis (European Pharmacopeia, current edition, chapter 5.3).

Specification: the lipasaemic activity must be comprised in the range of 10.0 LSU/mg - 14.0 LSU/mg.

Data management

In the event of the detection of coagulated blood (non-fluid blood with the presence of thickening), plasma with evident haemolysis (plasma with a reddish colour due to the presence of red blood cells) or the absence of the buffy coat (layer of leukocytes and platelets), the samples were nonetheless centrifuged and read.

The values generated by these samples were discarded, as coagulation and haemolysis are indicative of a sample collected incorrectly and the absence of the characteristic white layer of leukocytes and platelets is indicative of an anomalous separation of the blood. The values obtained from these samples were replaced in the spreadsheet by the mean of the other values belonging to the same group.

It was not permitted to exclude outlier values unless an evident problem with the sample was detected as indicated above.

Acceptability criteria for the results:

The analysis is deemed valid if:

1) The estimated activity is comprised between 80 and 125% of the known activity (Rel. To Ass.)
2) The confidence interval of the estimated activity is between 64 and 156% (Rel. To Est.)
3) The correlation coefficient (r) is $\geq 0.80$;
4) The p-value for the REGRESSION is "significant" (at least one asterisk). If this criterion is not satisfied, it is not possible to assign a confidence interval.

Validation operating procedure

For the purpose of validating the analytical method for the determination of the lipasaemic activity of Sulodexide, the following validation tests were performed:

☐ Substrate-system specificity;
☐ Specificity relative to the animals;
☐ Repeatability;
☐ Intermediate precision;
☐ Accuracy.

As this was an in vivo biological assay, the validation tests were selected in such a way as to ensure an adequate accuracy and precision in the implementation of the method, while at the same time abiding by the requests of regulatory agencies regarding a responsible and limited use of animals.

Specificity

Introduction and Procedure

Specificity expresses the ability of an analytical method to unequivocally determine the analyte in the presence of foreign substances that may be present in the material to be analysed or in the sample.

As this was an in vivo biological assay, in order to demonstrate the specificity of the method of determining the lipasaemic activity, the following were evaluated:

1) Substrate-System specificity
2) Animal specificity

Criterion of acceptability of the results

1) The Substrate-System specificity was verified through an assessment of the optical density of the lipid substrate.

2 ml of saline solution and 1 ml of lipid substrate were placed in a cuvette; the absorbance of this solution was tested at a wavelength of 600 nm after zeroing the instrument against air.

The reading must be comprised between 0.725 and 0.775 UAbs.

2) The animal specificity was evaluated by administering 1 ml of saline solution (placebo) to one animal and verifying that the A% obtained was a higher value than the one obtained with the administration of the standard solution 2 LSU/ml.

Repeatability

Procedure

The repeatability of the method was evaluated by performing three replicate determinations of lipasaemic activity on a lot of Sulodexide. Two replicates were performed by the same operator, whereas a third replicate was performed by a different operator.

Criterion of acceptability of the results

The Coefficient of Variation or Relative Standard Deviation over three replicate determinations of lipasaemic activity must be less than or equal to 20%. As this was an in vivo biological assay, the criterion of acceptability of the result was internally defined as acceptable.

Intermediate precision

Introduction

The intermediate precision expresses the variability normally present within a same laboratory, due mainly to different operators, different equipment, different days of testing, etc.

Procedure

In order to determine this parameter, a second operator, different from the one who performed the first repeatability replicate, analysed, on a different day, the same sample of Sulodexide as used for the repeatability tests according to the procedure described in the paragraph, determining in turn a value of lipasaemic activity.

Criterion of acceptability of the results

The Coefficient of Variation or Relative Standard Deviation between the two values of lipasaemic activity obtained must be less than or equal to 20%. As this was an in vivo biological assay, the criterion of acceptability of the result was internally defined as acceptable.

Accuracy

Criterion of acceptability of the results

The method is considered accurate if the mean percentage reduction (A%) relative to the 4 LSU/ml concentration of the standard of Sulodexide is > 50%. As this was an in vivo biological assay, the criterion of acceptability of the result was internally defined as acceptable.

**Summary of results**

At the end of the verification, a validation report was prepared, in which the result of the validation was briefly described.

Specificity

The method is specific, since:
1) the specificity of the substrate-system was demonstrated; i.e. the absorbance of the saline solution-substrate mixture is comprised in the range of 0.725 - 0.775.

**Table 2**

| SUBSTRATE-SYSTEM SPECIFICITY | | |
|---|---|---|
| **Replicates** | **ABS** | **Acceptability criterion** |
| 1 | 0.755 | |
| 2 | 0.753 | 0.725 - 0.775 |
| 3 | 0.756 | |

2) the animal specificity was demonstrated, i.e. when 1 ml of saline solution (placebo) was administered to an animal it was demonstrated that the A% is a greater value than the one obtained with the administration of the 2 LSU/ml solution.

**Table** 3

| ANIMAL SPECIFICITY | | | |
|---|---|---|---|
| Replicates | A% Placebo | A% STD 2 LSU/ml | Acceptability criterion |
| 1 | 92.39 | 87.25 | |
| 2 | 97.23 | 81.30 | A% placebo > A5 2 LSU/ml |
| 3 | 95.75 | 85.31 | |

**Repeatability**

The method is repeatable, since the coefficient of variation or relative standard deviation evaluated for the three replicate determinations of lipasaemic activity is $\leq 20\%$ (Table 4).

**Table 4**

| Replicates | Lipasaemic activity (LSU/mg) |
|---|---|
| 1 | 12.7 |
| 2 | 11.8 |
| 3 | 13.4 |
| Media | 12.6 |
| Std Deviation | 0.80 |
| Coeff. of Variation % | 6.3% |
| Acceptability criterion | $CV \leq 20\%$ |

**Accuracy**

The method is accurate since the mean percentage reduction (A%) relative to the 4 LSU/ml concentration of the standard of Sulodexide is > 50% (Table 5).

Table 5

| Accuracy | | |
|---|---|---|
| Replicates | A% 4 LSU/ml | Acceptability criterion |
| 1 | 64.20 | |
| 2 | 66.49 | > 50%. |
| 3 | 67.42 | |

The tests conducted demonstrate that the analytical method for determining lipasaemic activity in the active substance Sulodexide is correctly applied by the operators who conducted the validation of the method.

Therefore, an adequate precision of the biological test is assured, as it has demonstrated to be precise, repeatable, specific and accurate.

**Claims**

1. An in vitro method for evaluating the lipasaemic activity of a glycosaminoglycan-based compound comprising a heparin fraction, said method comprising the steps of:

   a) providing a lipid substrate;
   b) placing in contact with the lipid substrate:

   - a plurality of biological samples isolated from a first group of individuals to which a first concentration of the compound had been administered,
   - a plurality of biological samples isolated from a second group of individuals to which a second concentration of the compound, different from the first concentration of the compound, had been administered, and
   - optionally, a plurality of biological samples isolated from a third group of individuals to which a third concentration of the compound, different from the first and second concentrations of the compound, had been administered, in order to obtain at least four plasma-substrate systems obtained from the tested sample;

   c) placing in contact with the lipid substrate:

   - a plurality of biological samples isolated from a fourth group of individuals to which a first concentration of the compound with a known lipasaemic activity (lipasaemic units (LSU) /mg) had been administered (standard compound),
   - a plurality of biological samples isolated from a fifth group of individuals to which a second concentration of the standard compound, different from the first concentration of the standard compound, had been administered, and
   - optionally, a plurality of biological samples isolated from a sixth group of individuals to which a third concentration of the standard compound, different from the first and second concentrations of the standard compound, had been administered, in order to obtain at least four plasma-substrate systems obtained from the reference standard;

   d) reading the optical density of the plasma-substrate systems obtained from the tested sample and of the plasma-substrate systems obtained from the reference standard in steps b) and c) at a wavelength comprised between 550 and 650 nm after a time comprised between 0 and 2 minutes (T0) from the start of step b) and of step c);
   e) reading the optical density of the plasma-substrate systems obtained from the tested sample and of the plasma-substrate systems obtained from the reference standard in steps b) and c) at a wavelength comprised between 550 and 650 nm after a time comprised between 15 and 40 minutes (Tf) from the start of step a);
   f) evaluating the percentage reduction in the optical density (A%) of each plasma-substrate system obtained from the tested sample and of each plasma-substrate system obtained from the reference standard using the following formula:

$$A\% = (\text{Absorbance Tf} / \text{Absorbance T0}) \times 100,$$

wherein the A% values of the plasma-substrate system obtained from the reference standard are used to prepare a standard dose-response curve, wherein the dose is the concentration in LSU/ml of the standard compound administered and A% is the response,

wherein the A% values of the plasma-substrate systems obtained from the tested sample are used to prepare a dose-response curve, wherein the dose is the concentration in LSU/ml of compound administered and A% is the response,

wherein the lipasaemic activity of the compound is extrapolated by interpolating the A% values from the dose-response curve of the sample with the A% values of the standard dose-response curve,

wherein steps b) and c) are carried out at a temperature comprised between 36 and 40°C and wherein the individual had received the compound or the standard compound in an amount comprised between 1 and 10 lipasaemic units/ml (LSU/ml).

2. The method according to claim 1, wherein steps b) and c) are carried out at a temperature comprised between 37 and 38°C.

3. The method according to claim 1 or 2, wherein step a) comprises the following sub-steps:

   a1) preparing an aqueous phase;
   a2) preparing an oily phase;
   a3) mixing the aqueous phase obtained in sub-step a1) with the oily phase obtained in sub-step a2) using a homogeniser to obtain the lipid substrate; and
   a4) optionally sonicating the lipid substrate in step a3).

4. The method according to any one of claims 1-3, wherein the first concentration of the compound and of the standard compound is comprised between 0.5 and 4 LSU/ml, the second concentration of the compound and of the standard compound is comprised between 1 and 6 LSU/ml and the third concentration of the compound and of the standard compound is comprised between 4 and 10 LSU/ml.

5. The method according to claim 4, wherein the first concentration of the compound and of the standard compound is comprised between 1 and 3 LSU/ml; the second concentration of the compound and of the standard compound is comprised between 2 and 5 LSU/ml and the third concentration of the compound and of the standard compound is comprised between 6 and 9 LSU/ml.

6. The method according to any one of claims 1-5, wherein each group of individuals to which the compound or the standard compound had been administered comprises at least two individuals; it preferably comprises a number of individuals comprised between four and eight, more preferably comprised between five and six individuals.

7. The method according to any one of claims 1-6, wherein the compound and the standard compound are administered to the individuals by parenteral administration, preferably by injection.

8. The method according to any one of claims 1-7, wherein the individuals are animals, preferably rats.

9. The method according to claim 8, wherein the compound and the standard compound are administered to the individuals by injection into the caudal vein.

10. The method according to any one of claims 1 to 9, wherein the compound and the standard compound are Sulodexide.

11. The method according to any one of claims 1-10, wherein the biological samples are blood samples, optionally treated to obtain plasma.

12. The method according to claim 11, wherein the biological samples were isolated from the individuals by collecting blood from the abdominal aorta.

Europäisches
Patentamt
European
Patent Office
Office européen
des brevets

# EUROPEAN SEARCH REPORT

Application Number

EP 22 16 8828

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | JUN-PIL JEE ET AL: "Simplified analysis of lipoprotein lipase activity: Evaluation of lipasemic activity of low molecular weight heparin in rats", ARCHIVES OF PHARMACAL RESEARCH, PHARMACEUTICAL SOCIETY OF KOREA, HEIDELBERG, vol. 35, no. 6, 30 June 2012 (2012-06-30), pages 1107-1114, XP035078994, ISSN: 1976-3786, DOI: 10.1007/S12272-012-0619-4 * abstract; figure 7 * * page 1109, column 1, paragraph 2 - column 2, paragraph 2 * * page 1113, column 1, paragraph 1 * | 1-12 | INV. C12Q1/44 |
| A | Anonymous: "Lipoprotein Lipase Enzyme Activity Assay Validation and Clinical Assessment", , 11 July 2019 (2019-07-11), XP055962479, Retrieved from the Internet: URL:https://clinicaltrials.gov/ct2/show/NCT02656095 [retrieved on 2022-09-19] * the whole document * | 1-12 | TECHNICAL FIELDS SEARCHED (IPC) C12Q G01N |

The present search report has been drawn up for all claims

| Place of search | Date of completion of the search | Examiner |
|---|---|---|
| The Hague | 20 September 2022 | Gundlach, Björn |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)